# EUROPEAN PATENT SPECIFICATION

(11) **EP 1 325 329 B1**
(45) Date of publication and mention of the grant of the patent: **26.09.2012**
(21) Application number: 01975112.2
(22) Date of filing: 08.10.2001
(51) Int. Cl.: G01N 33/543, G01N 33/58

(54) **Assay method**
Testverfahren
Procédé d'éssai

(30) Priority: 11.10.2000 SE 0003662
(43) Date of publication of application: 09.07.2003
(73) Proprietor: Phadia AB, 751 37 Uppsala (SE)
(72) Inventor: MENDEL-HARTVIG, Ib, S-756 55 Uppsala (SE); ODELSTAD, Lena, S-755 98 Uppsala (SE)
(74) Representative: Lindgren, Anders
(86) International application number: PCT/SE2001/002185
(87) International publication number: WO 2002/031503

(56) References cited:
- EP-A1- 0 597 268
- EP-A1- 0 603 958
- EP-A2- 0 378 391
- EP-A2- 0 617 287
- WO-A1-00/00826
- WO-A2-00/08466
- US-A- 4 693 969
- US-A- 4 950 397
- US-A- 5 063 081
- US-A- 5 187 065
- US-A- 5 292 664
- GERT DOEKES ET AL.: 'Enzyme immunoassays for total and allergen specific IgE in population studies' OCCUPATIONAL AND ENVIRONMENTAL MEDICINE vol. 53, 1996, pages 63 - 70, XP002906812
- DATABASE WPI Week 8404, Derwent Publications Ltd., London, GB; AN 1984-020679, XP002906813 & JP 58 211 659 A (NISSUI SEIYAKU KK) 09 December 1983

## Description

### Field of the invention

The present invention relates to a method of quantitatively or semi-quantitatively determining an analyte in a sample, especially a high concentration analyte.

### Background of the invention

For qualitative and quantitative determination of an analyte in a sample, a socalled sandwich assay is often used, wherein two receptors directed against different epitopes of the analyte are incubated with a sample containing the analyte, one of the receptors being detectable, e.g. through a label conjugated thereto. In a heterogeneous assay format, the second receptor is immobilized (e.g. coupled) to a solid phase or provided with a binder component, such as biotin, capable of binding to the solid phase, such as an avidin- or streptavidin-coated solid phase.

Especially in case the analyte is present in the sample in a high concentration, it is customary to dilute the sample before performing the assay to avoid the use of large and often costly amounts of immobilized receptor and labelled receptor, respectively, or to avoid technical difficulties where large amounts of receptors cannot be used. Such dilution is not only laborious but also introduces an additional source of error into the assay.

There is therefore a need of an assay procedure that avoids the necessity of dilution.

### Summary of the invention

It is an object of the present invention to provide a method of performing a heterogeneous sandwich assay which permits the determination of even a high concentration analyte in a sample without the need to dilute the sample.

It is another object of the invention to provide a method of performing a heterogeneous sandwich assay which reduces the amounts of capturing and detection reagents used.

In one aspect of the present invention there is therefore provided a method of determining an analyte in a sample, especially a high concentration analyte found in concentrations> 1 nmole/litre, comprising the steps of:
a) contacting the sample with a specified amount of a receptor which binds specifically to the analyte to form an analyte/receptor complex, said specified amount of receptor being in excess of that required to bind all analyte in the sample,
b) isolating on a solid matrix such as a membrane strip, a minor fraction of the amount of receptor contacted with the analyte, wherein said fraction comprises both analyte/receptor complex and unreacted receptor,
c) detecting the amount of analyte/receptor complex in said isolated fraction, and
d) from the detected amount analyte/receptor complex, determining the concentration of analyte in the sample.

While it is preferred to use the method quantitative determination of analytes of interest, it may also be used for semi-quantitative and qualitative determinations.

### Detailed description of the invention

The essence of the present invention resides in binding all analyte present in a sample to an analyte-specific receptor, isolating a minor fraction of the analyte-receptor complex formed on a solid phase, detecting the amount of isolated analyte-receptor complex, and from this detected amount of analyte on the solid phase determining the total amount of analyte in the sample. According to the invention, this may be accomplished in various ways.

In one embodiment of method of the invention, all analyte is bound by contacting the analyte-containing sample with a solution containing an excess of a first receptor (R1) which in addition to affinity to the analyte has affinity to a ligand (L), whereupon a minor fraction of the analyte-receptor complex is bound to a solid phase having the ligand (L) immobilized thereto. This binding of the minor fraction may be achieved by either (i) using a limited (specified) amount of ligand (L) to extract a fraction of the analyte-receptor complex (and unreacted receptor), or (ii) by using a first receptor (R1) only a minor (specified) fraction of which is capable of binding to the ligand (L) to extract the desired fraction of the analyte-receptor complex (and unreacted receptor). In the latter case (ii), the amount of immobilized ligand (L) is usually in excess of the amount of the first receptor capable of binding to the ligand (L). The amount of analyte/receptor complex bound to the solid phase is then detected, usually by contacting the solid phase with a detecting agent in the form of a labelled binder for the analyte, such as a labelled second receptor (R2).

In the first case (i) above, the amount of immobilized ligand (L) that can bind to the analyte-specific receptor (R1) is a specified fraction of the amount of analyte-specific receptor (R1) contacted with the sample, and therefore the ratio of detected analyte on the solid phase to the total amount of analyte in the sample will correspond to the ratio of immobilized analyte-binding ligand (L) to the total amount of added receptor (R1), thereby permitting the analyte concentration in the sample to be calculated.

In the second case (ii) above, the amount of analyte-specific receptor (R1) that can bind to immobilized ligand (L) is a specified fraction of the total amount of receptor (R1), and therefore the ratio of detected analyte on the solid phase to the total amount of analyte in the sample will correspond to the ratio of analyte-specific receptor (R1) capable of reacting with ligand (L) to the total amount of receptor (R1), thereby permitting the analyte concentration in the sample to be calculated.

The term "receptor" as used herein refers to active analyte-binding receptor, and, where relevant, active ligand-binding receptor, respectively, and is not meant to include such receptor in an inactive or non-binding state. Likewise, the term receptor-binding ligand refers to active receptor-binding ligand and is not meant to include such ligand in an inactive or non-binding state.

The term "amount" as used herein usually means binding capacity. Thus, for example, when it is stated that the amount of analyte-specific receptor is in excess of the amount of analyte, it means that there is more analyte-specific receptor than necessary to bind all analyte. Usually, there is a 1:1 reaction ration between e.g. the analyte and the analyte-specific receptor, or between the analyte-specific receptor and the immobilized receptor-binding ligand. In such a case, the binding capacities of the respective species correspond to their molar amounts. Other reaction ratios are, however, also possible. For example, the immobilized ligand may be capable of binding more than one analyte-specific receptor.

In another embodiment of method of the invention, the sample is contacted with analyte-specific receptor (R1) provided both in solution and, in a minor fraction, immobilized to a solid phase, thereby permitting a minor fraction of analyte present in the sample to be bound to the solid phase. If the ratio of the amount of receptor (R1) in solution to the amount of immobilized receptor (R1) is known, the analyte concentration in the sample may be calculated from the detected amount of analyte bound to the solid phase.

It is readily seen that the above procedure gives the same effect as diluting the sample. In addition to the dilution step being avoided, which, of course, is of advantage to the operator, one obtains a considerable saving in reagents, i.e. both the reagent for capturing the analyte on the solid phase and the detecting agent, the latter often being costly. In this connection, it is also to be noted that in the assay of the invention, the reaction between analyte and receptor takes place in solution where almost all receptors are active rather than at a solid phase surface as in a corresponding conventional assay where only about 10-20% of immobilized receptor will react (Butler, J. E., et al, Molecular Immunology, Vol. 30, No. 13, pp. 1165-1175, 1993).

The required ratio between the total binding capacity of analyte-specific receptor contacted with the sample and (i) the binding capacity of receptor-binding ligand that is immobilized to the solid phase when this is limited, or (ii) the ligand-binding capacity of the analyte-specific receptor when this is limited, is readily determined by the skilled person depending *inter alia* on the particular analyte to be determined and the particular assay format used and may be chosen within wide limits. Usually, this ratio is from about 2:1 to about 1000:1, especially from about 5:1 to about 100:1, preferably more than about 10:1, more preferably more than about 20:1.

The excess of analyte receptor relative to the amount of analyte in the sample is also readily determined by the skilled person for each specific case.

The receptor contacted with the sample is usually of the dual receptor or bireactive binder type having one part that specifically binds to the analyte and another part which specifically binds to the ligand immobilized on the solid phase surface. The analyte binding part may, for example, be an antibody (monoclonal or polyclonal) or an active fragment thereof (including recombinant antibodies and fragments) or nucleic acids whereas the ligand-binding part may be one member of a specific binding pair. Exemplary such specific binding pairs include immunological binding pairs, such as antigen-antibody and hapten-antibody, biotin-avidin or -streptavidin, lectin-sugar, hormone-hormone receptor, and nucleic acid duplex. For example, the solid phase may have streptavidin immobilized thereto, and the receptor for the analyte may be biotinylated. To avoid immunoprecipitation at high analyte concentrations, it may be preferable to use monovalent receptors.

While the analyte preferably is a molecule present in concentrations >1 nmole/litre in a sample, the analyte may, of course, be any substance for which there exists a naturally occurring analyte-specific binding member or for which an analyte-specific binding member can be prepared.

Analyte that has been captured by the solid phase is usually detected by reaction with a labelled specific binder for the analyte. Such a labelled binder may be a conjugate comprising a detectable label covalently or non-covalently attached to the specific binding member, "label" referring to any substance which is capable of producing a signal that is detectable by visual or instrumental means, particularly a fluorophore or chromophore.

The sample is usually of biological origin, for example blood (serum, plasma, whole blood), saliva, tear fluid, urine, cerebrospinal fluid, sweat, etc. The invention is, of course, also applicable to other types of samples, such as fermentation solutions, reaction mixtures, etc. Especially, however, the sample is an undiluted serum or whole blood sample.

While the present invention is generally applicable, it may advantageously be used in solid phase assays of the immunochromatograpic type. Such assays use a device comprising a plate-shaped flow matrix of bibulous material, usually a membrane strip, such as of cellulose nitrate or glass fiber, in which liquid can be transported laterally (i.e. in the plane of the strip) by capillary forces in the membrane. The membrane usually has a sample application zone, and a detection (or reaction) zone downstream of the sample application zone. In the detection zone, usually a capturing reagent for the analyte is immobilized. To conduct an assay, the application zone is contacted with the liquid sample to be assayed for the analyte of interest. The device is maintained under conditions sufficient to allow capillary action of liquid to transport the analyte of interest, if present in the sample, through the membrane strip to the detection zone where the analyte is captured. The capillary liquid flow is usually insured by an absorbing pad or the like at the downstream end of the strip. A detection reagent, usually labelled, is then added upstream of the detection zone and interacts with captured analyte in the detection zone, and the amount of captured analyte is measured. Often, the detection reagent is pre-deposited in or on the membrane strip, e.g. in the form of diffusively movable particles containing fluorophoric or chromogenic groups, either upstream of the sample application zone or between the sample application zone and the detection zone.

In an immunochromatographic assay which may be used in the invention, the receptor is added to the sample either before applying the sample to the membrane strip, or may be pre-deposited in or on the membrane strip upstream of the detection zone.

A test kit for carrying out the method of the invention may comprise such a membrane having (i) immobilized in or on the membrane a ligand which binds specifically to the receptor, and (ii) dissolvably pre-deposited in or on the membrane a specified amount of analyte-specific receptor. The amount of the ligand on the solid phase member is less, and usually considerably less than that required to bind the specified amount of the receptor substance.

In another embodiment of test kit, only a fraction of the analyte-specific receptor is capable of binding to the immobilized ligand. Such a kit may comprise (i) immobilized in or on a membrane a ligand which binds specifically to the receptor, and (ii) dissolvably pre-deposited in or on the membrane a specified amount of analyte-specific receptor substance, only a specified fraction of which is capable of binding to the immobilized ligand.

Still another embodiment of test kit may comprise (i) dissolvably pre-deposited in or on a membrane a first specified amount of analyte-specific receptor substance, and (ii) immobilized in or on the membrane a second specified amount of the analyte-specific receptor substance.

In an alternative embodiment, the solid phase is a solid phase well, such as a microtiter plate well. Such of test kit may comprise a solid support having one or more wells with the second amount of analyte binding receptor immobilized therein and with the first amount of analyte-binding receptor dissolvably pre-deposited in the well or in close contact with the well.

In the following, the invention will be illustrated in more detail by a specific non-limiting Example.

### EXAMPLE 1

### Immunoassay for C-reactive protein (CRP) in undiluted serum samples Measuring range 10 - 200 mg/l

### Principle

Sample is mixed with biotinylated anti-CRP-fab in excess and the mixture is applied to a test strip having a deficient amount of streptavidin in the reaction zone. After an intermediate wash, anti-CRP fluorophore-conjugate is added and after a wash, conjugate that has bound to the reaction zone is measured. Since only a small part of the biotinylated anti-CRP-fab can bind to the reaction zone the consumption of the fluorophore conjugate is reduced considerably.

### Test strips

5 x 48 mm nitrocellulose membranes (Whatman, porosity 8 µm) on a polyester backing were used. The strips had a sample application zone at one end and a downstream reaction zone with immobilized streptavidin in an amount capable of binding approximately 6% of biotinylated anti-CRP added in the assay procedure.

### Samples

CRP-containing samples of varying CRP concentration were prepared from a 200 mg/l of recombinant CRP (Fitzgerald) in hCRP depleted serum.

### Procedure

15 µl of biotinylated anti-CRP-fab (monovalent fab-fragment of monoclonal antibody) and 15 µl of CRP-containing serum were mixed and the mixture was applied to the application zone of the membrane strip. The amount of biotinylated anti-CRP-fab was 3 µg per test strip, which is a 2 x molar excess of anti-CRP in relation to the standard 200 mg/l CRP. After an intermediate wash with 15 µl of test buffer (50 mM borate buffer pH 8.0, 3% BSA, 5% sucrose, 0.15 M NaCl, 0.005% CaCl₂, 0.05% NaN₃), 15 µl of detection conjugate solution [3 µg of anti-CRP monoclonal antibody (Fitzgerald) coupled to 0.1 µm TransFluoSpheres-SO₄/CHO (633/720 nm) (Molecular Probes Inc.), the above test buffer] were added , followed by wash with 2 x 15 µl of test buffer. The fluorescence of the strip was then measured. The results are shown in Table 1 below.

**Table 1**

| **CRP conc. (mg/l)** | **Peak area obtained (V x mm)** |
|---|---|
| 0 | 0.08 |
| 0 | 0.07 |
| 10 | 2.56 |
| 10 | 2.50 |
| 30 | 3.62 |
| 30 | 4.01 |
| 100 | 5.24 |
| 100 | 4.87 |
| 200 | 6.28 |
| 200 | 5.82 |

### EXAMPLE 2 (comparative)

### Immunoassay for CRP in serum samples diluted 1/20 Measurement range 10 - 200 mg/ml

### Principle

Sample is diluted in test buffer and applied to test strips having an excess of anti-CRP in the reaction zone. Anti-CRP fluorophore-conjugate is then added followed by a wash, whereupon conjugate that has bound to the reaction zone is measured. Sample dilution is necessary to avoid unreasonably large amounts of anti-CRP in the reaction zone as well as in the detection conjugate.

### Test strips

5 x 48 mm nitrocellulose membranes (Whatman, porosity 8 µm) on a polyester backing were used. The strips had a sample application zone at one end and a downstream reaction zone with 2.6 µg immobilized anti-CRP monoclonal antibody (Fitzgerald), which is a 13 x molar excess in relation to a standard 10 mg/ml CRP serum.

### Samples

CRP-containing samples of varying CRP concentration were prepared from a 200 mg/l of recombinant CRP (Fitzgerald) in hCRP depleted serum.

### Procedure

15 µl of CRP-containing serum diluted 1/20 in test buffer (50 mM borate buffer pH 8.0, 3% BSA, 5% sucrose, 0.15 M NaCl, 0.005% CaCl₂, 0.05% NaN₃) were applied to the application zone of the membrane strip. Then, 15 µl of detection conjugate solution [anti-CRP monoclonal antibody (Fitzgerald) coupled to 0.1 µm TransFluoSpheres-SO₄/CHO (633/720 nm) (Molecular Probes Inc.), the above test buffer] were added, the amount of anti-CRP conjugate being 3 µg per test strip which was a 15 x molar excess in relation to the highest standard value. The conjugate addition was followed by a wash with 15 µl of test buffer. The fluorescence of the strip was then measured. The results are shown in Table 2 below.

**Table 2**

| **CRP conc. (mg/l)** | **Peak area obtained (V x mm)** |
|---|---|
| 0 | 0.41 |
| 0 | 0.60 |
| 10 | 7.51 |
| 10 | 7.130 |
| 20 | 8.86 |
| 20 | 9.42 |
| 40 | 11.97 |
| 40 | 10.67 |
| 80 | 11.70 |
| 80 | 12.91 |
| 200 | 14.27 |
| 200 | 14.16 |

The above Examples 1 and 2 thus demonstrate that it is possible to run an assay on undiluted high concentration samples without using huge amounts of reagents when using the methodology of the present invention.

## Claims

1. A method of determining an analyte in a sample comprising the steps of:
a) contacting the sample with a specified amount of a receptor which binds specifically to the analyte to form an analyte/receptor complex, said specified amount of receptor being in excess of that required to bind all analyte in the sample,
b) isolating on a solid matrix a minor fraction of the amount of receptor contacted with the analyte, wherein said fraction comprises both the analyte/receptor complex and unreacted receptor,
c) detecting the amount of analyte/receptor complex in said isolated fraction, and
d) from the detected amount analyte/receptor complex, determining the concentration of analyte in the sample.

2. The method according to claim 1 in which the analyte in the sample has a concentration of >1 nmole/litre.

3. The method according to claim 1 or claim 2 in which the sample is undiluted.

4. The method according to claims 1 to 3, wherein isolating said fraction of the amount of receptor contacted with the sample on the solid matrix comprises providing a solid matrix having binding sites for the receptor, and after contacting the sample, or an aliquot thereof, with a liquid phase containing the receptor, binding said fraction of receptor to the solid matrix.

5. The method according to claim 4, wherein the whole amount of receptor has reactivity towards said binding sites on the solid matrix, and the receptor-binding capacity of the solid matrix is less than the solid-matrix-binding capacity of receptor contacted with the sample.

6. The method according to claim 4, wherein only a fraction of the amount of receptor contacted with the sample has reactivity towards said binding sites on the solid matrix.

7. The method according to claims 1 to 3, wherein isolating said fraction of the amount of receptor on the solid matrix comprises contacting the sample with a specified amount of receptor, a fraction of which amount is immobilized to said solid matrix and the remaining amount of receptor being in a liquid phase.

8. The method according to any one of claims 1 to 6, wherein the receptor comprises a first part that binds specifically to the analyte, and a second part that binds to the solid matrix.

9. The method according to claim 8, wherein the solid matrix binding part of the receptor comprises one member of a specific binding pair, and the other member of the binding pair is immobilized to the solid matrix.

10. The method according to any one of the preceding of claims, wherein in step c) the analyte/receptor complex is detected by a labelled detection reagent which binds specifically to the analyte.

11. The method according to any one of the preceding claims, wherein the ratio between said isolated fraction of the amount of active analyte-binding receptor and the total amount of active analyte-binding receptor contacted with the sample is in the range of from about 1:2 to about 1:1000, preferably from about 1:5 to about 1:100, particularly no more than about 1:20.

12. The method according to any one of the preceding claims, wherein said solid matrix binding sites for the receptor are immobilized in a reaction zone of a flow matrix, preferably a lateral flow matrix, such as a membrane strip.

13. The method according to any one of the preceding claims, wherein the receptor is an antibody or an immunoactive fragment thereof.

14. The method according to any one of the preceding claims, wherein the detection reagent is an antibody or an immunoactive fragment thereof.

15. The method according to any one of the preceding claims, wherein the detection reagent is labelled by a fluorophore or a chromophore.

16. The method according to any one of the preceding claims, wherein the specific binding pair is biotin-avidin or biotin-streptavidin.

17. The method according to any one of the preceding claims, wherein the sample is an undiluted serum sample.

18. The method according to any one of claims 1 to 16, wherein the sample is an undiluted whole blood sample.

## Patentansprüche

1. Verfahren zur Bestimmung eines Analyten in einer Probe mit den Schritten:
a) In-Kontakt-bringen der Probe mit einer festgelegten Menge eines Rezeptors, der sich an den Analyt spezifisch bindet, um einen Analyt/Rezeptor-Komplex zu bilden, wobei die festgelegte Menge des Rezeptors diejenige übersteigt, die erforderlich ist, den gesamten Analyt in der Probe zu binden,
b) Isolieren eines geringen Anteils der mit dem Analyt in Kontakt gebrachten Menge von Rezeptor auf einer festen Matrix, wobei der Anteil sowohl den Analyt/Rezeptor-Komplex als auch nicht in Reaktion getretenen Rezeptor aufweist,
c) Detektieren der Menge von Analyt/Rezeptor-Komplex im isolierten Anteil, und
d) anhand der detektierten Menge von Analyt/Rezeptor-Komplex erfolgendes Bestimmen der Konzentration von Analyt in der Probe.

2. Verfahren nach Anspruch 1, wobei der Analyt in der Probe eine Konzentration > 1 nmol/Liter hat.

3. Verfahren nach Anspruch 1 oder Anspruch 2, wobei die Probe unverdünnt ist.

4. Verfahren nach Anspruch 1 bis 3, wobei das Isolieren des Anteils der mit der Probe in Kontakt gebrachten Menge von Rezeptor auf der festen Matrix aufweist: Bereitstellen einer festen Matrix mit Bindungsstellen für den Rezeptor, und Binden des Anteils von Rezeptor an die feste Matrix nach In-Kontakt-bringen der Probe oder einer Aliquote davon mit einer den Rezeptor enthaltenden flüssigen Phase.

5. Verfahren nach Anspruch 4, wobei die gesamte Menge von Rezeptor Reaktivität gegenüber den Bindungsstellen auf der festen Matrix hat und das Rezeptorbindungsvermögen der festen Matrix kleiner als das Bindungsvermögen von mit der Probe in Kontakt gebrachtem Rezeptor an der festen Matrix ist.

6. Verfahren nach Anspruch 4, wobei nur ein Anteil der mit der Probe in Kontakt gebrachten Menge von Rezeptor Reaktivität gegenüber den Bindungsstellen auf der festen Matrix hat.

7. Verfahren nach Anspruch 1 bis 3, wobei das Isolieren des Anteils der Menge von Rezeptor auf der festen Matrix aufweist: In-Kontakt-bringen der Probe mit einer festgelegten Menge von Rezeptor, wobei ein Anteil dieser Menge an der festen Matrix immobilisiert wird und die restliche Menge von Rezeptor in einer flüssigen Phase vorliegt.

8. Verfahren nach einem der Ansprüche 1 bis 6, wobei der Rezeptor aufweist: einen ersten Teil, der sich spezifisch an den Analyt bindet, und einen zweiten Teil, der sich an die feste Matrix bindet.

9. Verfahren nach Anspruch 8, wobei der sich an die feste Matrix bindende Teil des Rezeptors ein Element eines spezifischen Bindungspaars aufweist und das andere Element des Bindungspaars an der festen Matrix immobilisiert wird.

10. Verfahren nach einem der vorstehenden Ansprüche, wobei im Schritt c) der Analyt/Rezeptor-Komplex durch ein markiertes Detektionsreagens detektiert wird, das sich spezifisch an den Analyt bindet.

11. Verfahren nach einem der vorstehenden Ansprüche, wobei das Verhältnis zwischen dem isolierten Anteil der Menge von aktivem, sich an den Analyt bindendem Rezeptor und der Gesamtmenge von aktivem, sich an den Analyt bindendem Rezeptor, die mit der Probe in Kontakt gebracht wird, im Bereich von etwa 1:2 bis etwa 1:1000, vorzugsweise von etwa 1:5 bis etwa 1:100 liegt und insbesondere höchstens etwa 1:20 beträgt.

12. Verfahren nach einem der vorstehenden Ansprüche, wobei die Bindungsstellen der festen Matrix für den Rezeptor in einer Reaktionszone einer Flow-Matrix, vorzugsweise einer Lateral-Flow-Matrix, z. B. eines Membranstreifens, immobilisiert werden.

13. Verfahren nach einem der vorstehenden Ansprüche, wobei der Rezeptor ein Antikörper oder ein immunaktives Fragment davon ist.

14. Verfahren nach einem der vorstehenden Ansprüche, wobei das Detektionsreagens ein Antikörper oder ein immunaktives Fragment davon ist.

15. Verfahren nach einem der vorstehenden Ansprüche, wobei das Detektionsreagens durch ein Fluorophor oder ein Chromophor markiert ist.

16. Verfahren nach einem der vorstehenden Ansprüche, wobei das spezifische Bindungspaar Biotin-Avidin oder Biotin-Streptavidin ist.

17. Verfahren nach einem der vorstehenden Ansprüche, wobei die Probe eine unverdünnte Serumprobe ist.

18. Verfahren nach einem der Ansprüche 1 bis 16, wobei die Probe eine unverdünnte Vollblutprobe ist.

## Revendications

1. Procédé pour déterminer un analyte dans un échantillon, comprenant les étapes consistant à :
a) mettre l'échantillon en contact avec une quantité spécifiée d'un récepteur qui se lie spécifiquement à l'analyte pour former un complexe analyte/récepteur, ladite quantité spécifiée de récepteur étant en excès par rapport à celle requise pour lier tout l'analyte dans l'échantillon,
b) isoler, sur une matrice solide, une fraction mineure de la quantité de récepteur en contact avec l'analyte, ladite fraction comprenant à la fois le complexe analyte/récepteur et du récepteur n'ayant pas réagi,
c) détecter la quantité de complexe analyte/récepteur dans ladite fraction isolée, et
d) à partir de la quantité détectée de complexe analyte/récepteur, déterminer la concentration d'analyte dans l'échantillon.

2. Procédé selon la revendication 1, dans lequel l'analyte dans l'échantillon a une concentration > 1 nanomole par litre.

3. Procédé selon la revendication 1 ou la revendication 2, dans lequel l'échantillon n'est pas dilué.

4. Procédé selon les revendications 1 à 3, dans lequel l'isolement de ladite fraction de la quantité de récepteur en contact avec l'échantillon sur la matrice solide comprend les opérations consistant à fournir une matrice solide ayant des sites de liaison pour le récepteur et, après mise en contact de l'échantillon, ou d'une aliquote de celui-ci, avec une phase liquide contenant le récepteur, à lier ladite fraction de récepteur à la matrice solide.

5. Procédé selon la revendication 4, dans lequel la quantité totale de récepteur a une réactivité vis-à-vis desdits sites de liaison sur la matrice solide, et la capacité de liaison au récepteur de la matrice solide est inférieure à la capacité de liaison à la matrice solide du récepteur en contact avec l'échantillon.

6. Procédé selon la revendication 4, dans lequel seule une fraction de la quantité de récepteur en contact avec l'échantillon a une réactivité vis-à-vis desdits sites de liaison sur la matrice solide.

7. Procédé selon les revendications 1 à 3, dans lequel l'isolement de ladite fraction de la quantité de récepteur sur la matrice solide comprend la mise en contact de l'échantillon avec une quantité spécifiée de récepteur, une fraction de cette quantité étant immobilisée sur ladite matrice solide et la quantité restante de récepteur étant dans une phase liquide.

8. Procédé selon l'une quelconque des revendications 1 à 6, dans lequel le récepteur comprend une première partie qui se lie spécifiquement à l'analyte, et une deuxième partie qui se lie à la matrice solide.

9. Procédé selon la revendication 8, dans lequel la partie se liant à la matrice solide du récepteur comprend un membre d'une paire de liaison spécifique, et l'autre membre de la paire de liaison est immobilisé sur la matrice solide.

10. Procédé selon l'une quelconque des revendications précédentes, dans lequel, dans l'étape c), le complexe analyte/récepteur est détecté par un réactif de détection marqué qui se lie spécifiquement à l'analyte.

11. Procédé selon l'une quelconque des revendications précédentes, dans lequel le rapport de ladite fraction isolée de la quantité de récepteur se liant à l'analyte actif à la quantité totale du récepteur se liant à l'analyte actif en contact avec l'échantillon est situé dans la plage allant d'environ 1:2 à environ 1:1000, de préférence d'environ 1:5 à environ 1:100, en particulier n'est pas supérieur à environ 1:20.

12. Procédé selon l'une quelconque des revendications précédentes, dans lequel lesdits sites de liaison à la matrice solide pour le récepteur sont immobilisés dans une zone réactionnelle d'une matrice à écoulement, de préférence une matrice à écoulement latéral, tel qu'une bandelette de membrane.

13. Procédé selon l'une quelconque des revendications précédentes, dans lequel le récepteur est un anticorps ou un fragment immunoactif de celui-ci.

14. Procédé selon l'une quelconque des revendications précédentes, dans lequel le réactif de détection est un anticorps ou un fragment immunoactif de celui-ci.

15. Procédé selon l'une quelconque des revendications précédentes, dans lequel le réactif de détection est marqué par un fluorophore ou un chromophore.

16. Procédé selon l'une quelconque des revendications précédentes, dans lequel la paire de liaison spécifique est la biotine-avidine ou la biotine-streptavidine.

17. Procédé selon l'une quelconque des revendications précédentes, dans lequel l'échantillon est un échantillon de sérum non dilué.

18. Procédé selon l'une quelconque des revendications 1 à 16, dans lequel l'échantillon est un échantillon de sang entier non dilué.
